# FASCICULE DE BREVET EUROPEEN

(11) **EP 0 651 784 B1**
(45) Date de publication et mention de la délivrance du brevet: **17.04.2002**
(21) Numéro de dépôt: 93913171.0
(22) Date de dépôt: 22.06.1993
(51) Int. Cl.: C12N 1/19, C12N 15/81, C12N 15/52

(54) **SOUCHES DE LEVURE EXPRIMANT LE GENE DE LA LDH LACTIQUE, ET VECTEURS UTILISABLES POUR L'OBTENTION DESDITES SOUCHES**
HEFEART DIE DAS GEN DER LAKTATDEHYDROGENASE DER MILCH EXPRIMIERT, UND VERWENDBARE VEKTOREN ZUR HERSTELLUNG DIESER ARTEN
YEAST STRAINS EXPRESSING A LACTIC LACTICODESHYDROGENASE GENE AND VECTORS USEFUL IN PRODUCING SAID STRAINS

(30) Priorité: 23.06.1992 FR 9207632
(43) Date de publication de la demande: 10.05.1995
(73) Titulaire: INSTITUT NATIONAL DE LA RECHERCHE AGRONOMIQUE, F-75341 Paris Cédex 07 (FR)
(72) Inventeur: DEQUIN, Sylvie, F-34000 Montpellier (FR); BARRE, Pierre, F-34980 S.-Gely-du-Fesc (FR)
(74) Mandataire: Orès, Bernard
(86) Numéro de dépôt international: FR9300618
(87) Numéro de publication internationale: WO9400554

(56) Documents cités:
- EP-A- 0 103 399
- APPLIED AND ENVIRONMENTAL MICROBIOLOGY, vol. 57, no. 8, August 1991, pages 2413-2417, Washington DC, US, S.F. KIM et al.: "Cloning and Nucleotide Sequence of the Lactobacillus casei Lactate Dehydrogenase Gene", le document en entier (cited in the application)
- idem
- DATABASE WPIL, Derwent Publications Ltd., London, GB, DATABASE WPIL, AN 91-373410, week 9151, & JP - A - 3251172 (MEIJI MILK PRODS KK) 8 novembre 1991, abrégé

## Description

L'Invention est relative à la construction de levures exprimant une fermentation mixte alcoolique-lactique.

Ces deux grands types de fermentation des sucres sont traditionnellement mis en oeuvre dans l'industrie agro-alimentaire : la fermentation alcoolique, dont sont responsables des levures du genre *Saccharomyces*, conduit pour l'essentiel à la formation d'éthanol et de CO₂ ; et la fermentation lactique (bactéries lactiques) conduit à la formation d'acide lactique.

La fermentation alcoolique et la fermentation lactique présentent des voies métaboliques pratiquement superposables jusqu'au pyruvate. A ce stade, cet intermédiaire est traité différemment par deux systèmes accepteurs finaux d'électrons. Dans la fermentation alcoolique, le pyruvate est décarboxylé en acétaldéhyde, ce dernier composé étant réduit en éthanol par le biais d'une alcool déshydrogénase ; dans la fermentation lactique, le pyruvate est réduit directement en lactate par le biais d'une lacticodéshydrogénase (LDH).

Les microorganismes utilisés en agro-alimentaire possèdent l'une ou l'autre de ces deux voies métaboliques et accomplissent de manière exclusive l'une ou l'autre de ces deux fermentations.

Les Inventeurs ont entrepris de construire une souche de levure pouvant accomplir à la fois la fermentation alcoolique et la fermentation lactique, ce qui résulte en une fermentation à bilan intermédiaire entre ces deux types.

Le principe de la construction réalisée par les Inventeurs a consisté à cloner le gène d'une lacticodéshydrogénase d'une bactérie lactique GRAS (*Lactobacillus casei*) et à faire exprimer ce gène chez *Saccharomyces* pour y installer, en bout de flux carboné, un système accepteur d'électrons devant concurrencer le système sauvage.

Une telle construction n'avait jamais été proposée dans l'art antérieur, car il était généralement admis que trois grands groupes d'obstacles pouvaient faire échec à son fonctionnement :
- une expression nulle ou insuffisante du gène bactérien chez *Saccharomyces ;*
- un fonctionnement non concurrentiel du système accepteur introduit ;
- une incompatibilité entre le fonctionnement du système et la viabilité et l'activité fermentaire de *Saccharomyces,* résultant par exemple d'un problème de translocation membranaire du lactate.

Or, de façon surprenante, les Inventeurs sont parvenus à obtenir un système viable et fonctionnel, et conduisant au détournement d'une part importante du flux carboné vers le lactate.

La présente Invention a pour objet des souches de levure caractérisées en ce qu'elles contiennent au moins une copie d'un gène codant pour une LDH de bactérie lactique, sous contrôle de séquences régulant l'expression dudit gène dans la levure.

Par "séquences régulant l'expression d'un gène" on entend des séquences de type promoteur et terminateur actives dans la levure. Les promoteurs et terminateurs de différents gènes peuvent être utilisés, associés dans des combinaisons différentes. On citera, à titre d'exemple non limitatif, les promoteurs et terminateurs, connus en eux-mêmes, des gènes alcool-déshydrogénase I (ADHI), phosphoglycérate kinase (PGK), et glycéraldéhyde-3-phosphate déshydrogénase (GAPDH).

L'invention comprend également la cassette d'expression obtenue en associant lesdites séquences de régulation et le gène de la LDH ; cette cassette peut être portée par un plasmide, ou intégrée dans l'ADN chromosomique de la levure hôte.

Selon un mode de réalisation préféré dela présente Invention, lesdites levures appartiennent au genre *Saccharomyces.*

Selon un autre mode de réalisation préféré dela présente Invention, le gène exprimé est celui de la LDH de *Lactobacillus casei*.

Pour que le gène intégré s'exprime dans la levure, le codon d'initiation GTG est préalablement modifié en ATG.

L'Invention englobe également des vecteurs d'expression, comprenant une cassette d'expression telle que définie ci-dessus, et utilisables pour l'obtention des souches de levure transformées conformes à l'Invention.

Ces vecteurs pourront être sélectionnés sur la base de la nature et de la force des éléments régulateurs qui entrent dans la cassette d'expression. On peut choisir les promoteurs et terminateurs décrits plus haut, ou toute autre séquence permettant de contrôler l'expression d'un gène dans la levure.

Un autre critère du choix des vecteurs réside dans le nombre de copies de ceux-ci, qui est conditionné par le choix de l'origine de réplication.

On peut choisir d'intégrer le gène de la LDH, muni de ses séquences de contrôle, dans le génome de la levure, auquel cas on pourra par exemple choisir un vecteur intégratif (YIp) ne possédant pas d'origine de réplication dans la levure ; il est également possible d'intégrer ce gène en utilisant d'autres techniques, par exemple la co-transformation.

S'il est préférable que le gène de la LDH soit porté par un plasmide, un choix peut être fait entre les vecteurs suivants :
. Vecteur réplicatif (YEp) à haut nombre de copies, possédant comme origine de réplication dans la levure une partie du plasmide 2µ endogène.
. Vecteur réplicatif (YRp) à haut nombre de copies, possédant comme origine de réplication une séquence ARS chromosomique.
. Vecteur réplicatif (YLp) linéaire à haut nombre de copies possédant des séquences télomériques comme origine de réplication.
. Vecteur réplicatif (YCp) à bas nombre de copies, possédant une séquence ARS chromosomique et une séquence centromérique.

Préférentiellement, les vecteurs conformes à l'Invention comportent également des marqueurs de sélection dans la levure, tels que des marqueurs d'auxotrophie : URA₃, LEU₂, HIS₃, TRP₁, ADE, etc ...et/ou des marqueurs de résistance à des antibiotiques (G418, hygromycine B, chloramphénicol, phléomycine), à des herbicides (sulfométuron methyl), au cuivre, etc...

Avantageusement, les vecteurs conformes à l'Invention sont des vecteurs navette, possédant également une origine de réplication bactérienne et un marqueur de sélection dans une bactérie (par exemple, gène de résistance à un antibiotique).

Les plasmides conformes à l'Invention, portant le gène codant pour la LDH d'une bactérie lactique peuvent être introduits dans toutes souches de levure, par différentes techniques de transformation.

Parmi les techniques de transformation les plus courantes, on citera la technique des protoplastes, la technique de perméabilisation aux sels de lithium et l'électroporation.

Il est possible de moduler le taux d'expression du gène codant pour la LDH, et en conséquence le rapport éthanol-lactate, en jouant notamment sur le nombre de copies du gène LDH introduites dans la levure, et/ou sur la force des éléments de régulation qui y sont associés.

La construction de différentes souches conformes à l'invention, exprimant la LDH à des degrés divers, peut être réalisée selon l'utilisation recherchée, qui détermine le rapport éthanol/lactate souhaité.

Dans tous les cas, le procédé de cette construction comprend les étapes suivantes :
- construction d'une cassette d'expression comprenant le gène LDH et des éléments régulateurs de force variable, selon le rapport éthanol/lactate souhaité ;
- introduction de cette cassette soit en monocopie, soit en multicopie (selon le rapport éthanol/lactate souhaité) dans la levure.

Les souches de levure conformes à l'invention trouvent de nombreuses applications dans l'agro-alimentaire. Elles sont utilisables partout où une fermentation alcoolique doit s'accompagner d'une acidification biologique (par exemple élaboration de cidre à partir de pommes insuffisamment acides, élaboration de pâtes à pain dites acides, produits lactés type kéfir ...), ou d'un abaissement de son rendement en éthanol.

Ceci est particulièrement intéressant dans le domaine de l'oenologie :
- pour palier au manque d'acidité d'un nombre de plus en plus important de vins, notamment dans les régions chaudes ;
- pour pouvoir bénéficier des avantages de la fermentation malolactique (stabilisation biologique du produit) même lorsqu'elle conduit à une désacidification trop poussée, en compensant par une acidification ;
- pour produire des vins ou des boissons à teneur allégée en éthanol.

En outre, en poussant la déviation de la fermentation alcoolique à son extrême, des levures *Saccharomyces* transformées conformément à l'Invention permettraient de remplacer les bactéries pour réaliser la fermentation lactique. Ces levures présenteraient les avantages suivants : insensibilité aux phages, croissance en milieu pauvre en nutriments, croissance en milieu acide, croissance à température plus basse. Elles pourraient également être mises en oeuvre pour la production industrielle d'acide lactique.

La présente Invention sera mieux comprise à l'aide du complément de description qui va suivre, qui se réfère à des exemples de construction de souches de levure transformées conformes à l'Invention, ainsi qu'à la démonstration de leur activité fermentaire.

Il va de soi toutefois que ces exemples sont donnés uniquement à titre d'illustration de l'objet de l'Invention, dont ils ne constituent en aucune manière une limitation.

### EXEMPLE 1 : CLONAGE DU GENE DE LA L-LDH DE L. CASEI

### A) Construction d'une banque d'ADN de L. casei dans E. coli

### a) Extraction de l'ADN de L. casei

La souche *Lactobacillus casei* ATCC 393 a été utilisée. Elle a été cultivée sur milieu MRS dont la composition est la suivante : polypeptone 10 g/l, extrait de levure 5 g/l, extrait de viande 10 g/l, glucose 2 g/l, phosphate dipotassique 2 g/l, acétate de sodium 5 g/l, citrate d'ammonium 2 g/l, sulfate de magnésium 0,2 g/l, sulfate de manganèse 0,05 g/l, tween 80 1ml/l.

15 ml de milieu MRS sont ensemencés par *L. casei* et incubés à 37°C pendant une nuit. 500 ml de même milieu sont ensemencés par les 15 ml de préculture ainsi obtenus, et incubés à 37°C avec légère agitation jusqu'à une DO (550 nm) de 3,9.

L'ADN est extrait selon LERCH et al. [Yeast, 7:253-263, (1989]) et purifié sur gradient de chlorure de césium en présence de bromure d'éthidium [MANIATIS et al., Molecular cloning: A Laboratory Manual, Cold Spring Harbor Laboratory (1989).

Le bromure d'éthidium est extrait à l'isobutanol (2 fois) et à l'alcool isoamylique (2 fois). Après dilution par 2 volumes d'eau, l'ADN est précipité par 6 volumes d'éthanol.

Le précipité est récupéré à la baguette et dissous dans 2 ml de TE (Tris 10 mM EDTA 1 mM pH 7.5).

La concentration de l'ADN en solution est déterminée par mesure de la DO à 260 nm.

### b) Digestion de l'ADN de L. casei

60 µg d'ADN de *L. casei* sont digérés partiellement par 8 unités d'enzyme Sau3A pendant 40 mn à 37°C.

Le mélange est extrait au phénol, phénol-chloroforme, chloroforme et précipité à l'alcool.

Après centrifugation, l'ADN est repris dans 300 µl de TE et les fragments séparés sur gradient de sucrose pendant 15 heures à 25000 rpm.

Des fractions de 0,5 ml sont récoltées et analysées sur gel d'agarose 0,8%. La fraction contenant des fragments de 2 à 4 kb est dialysée contre du TE pendant 4 heures.

### c) Ligation de l'ADN digéré au vecteur pUC18

L'ADN digéré est ensuite ligué au vecteur pUC18Bam HI déphosphorylé (Appligène), pour la réalisation de la banque, dans les conditions suivantes :

| | |
|---|---|
| pUC18BdP | 5 µl (250 ng) |
| ADN digéré | 10 µl (1 µg) |
| APPLIGENE Tp Ligation 5X | 10 µl |
| APPLIGENE Ligase | 5 µl |
| Eau | 20 µl |

Le mélange est incubé pendant 18 h à 14°C

### d) Transformation de E. coli

La souche de *E. coli* DH5α (GIBCO BRL) de génotype : F⁻ ; endA1 ; hsdR17(K⁻, mK⁻) ; supE44 ; Thi-1 ; λ- ; recA1 ; gyrA96 ; relAl, a été utilisée.

Les protocoles utilisés pour la préparation des bactéries compétentes et ceux pour la transformation sont décrits par HANAHAN [In DNA cloning, vol. 1 DM Glover (ed) IRL Press, 109-135 (1985)].

Le mélange de ligation a été utilisé pour transformer les bactéries compétentes DH5α. Les colonies obtenues ont été sélectionnées sur boîte LB (bactotryptone 10g/l, extrait de levure 5g/l, Nacl 10g/l) + ampicilline (50µg/ml). 92% des clones avaient un insert de la taille attendue (2-4 kb).

### B. - Amplification d'un Fragment du gène LDH par PCR

A partir de la séquence protéique de la L-LDH de *Lactobacillus casei* publiée par HENSEL et al. [Eur. J. Biochem 134:503-511, (1983)], deux mélanges d'oligonucléotides ont été synthétisés. L'amorce 4122 est dérivée des acides aminés 5 à 10 : Asp-Lys-Asp-His--Gln-Lys et l'amorce 5036 est dérivée des acides aminés 262 à 267 : Tyr-Met-Asp-Gly-Gln-tyr (Kim et al. 1991).
4122 : 5'-GA(C,T)-AA(G,A)-GA(C,T)-CA(C,T)-CA(G,A)-A-(A)-3' 5036 :

5'-TA-(C,T)TG-(ACTG)CC-(G,A)TC-CAT-(G,A)TA-3'

Les 2 amorces ont été utilisées pour amplifier un fragment du gène de la L.LDH de *L. casei* à partir de l'ADN total isolé de cette souche ; la taille du fragment amplifié est de 785 paires de bases. L'amplification a été réalisée comme suit :

| | |
|---|---|
| Amorce 4122 | 5 µl (100 pmoles) |
| Amorce 5036 | 7,2 µl (100 pmoles) |
| Taq Buffer 10X | 10 µl |
| Taq (BECKMAN) 5u/µl | 0,5 µl |
| dNTP (2 mM) | 10 µl |
| ADN *L. casei* (10ng/µl) | 10 µl (100 ng) |
| Eau | 57 µl |

Taq buffer 10X : Tris HCl pH 8,3 100mM, KCl 500mM, MgCl₂ 15mM
Conditions d'amplification : 1 mn 94°C, 1mn 55°C, 2 mn 72°C pendant 30 cycles, sur amplificateur PHC2 TECHNE.

Le produit d'amplification est analysé sur gel d'agarose 1,5% et la bande correspondante (785 bp) prélevée. L'ADN est élué sur colonne MILLIPORE ULTRAFREE, purifié par extractions classiques au phénol précipité à l'alcool, puis centrifugé et repris dans du tampon TE.

### C. - Criblage de la banque

### a) Marguage du fragment de 785 bp

Le fragment amplifié est marqué au ³²P par la technique multiprime, à l'aide du kit "Rapid hybridization system multiprim" (AMERSHAM). L'ADN est dénaturé à 100°C pendant 3 mn puis refroidi brutalement dans la glace. Le marquage est effectué dans les conditions suivantes :

| | |
|---|---|
| ADN 785 bases | 12 µl (100 ng) |
| tampon de marquage | 10 µl |
| hexanucléotides | 5 µl |
| dCTP (32P) (3000 ci/mmoles) | 4 µl |
| Klenow | 2 µl |
| H2O | 17 µl |

pendant 2 heures à 37°C.

La sonde marquée est séparée des nucléotides par filtration sur colonne NENSORB (NEN). La radioactivité des fractions éluées est déterminée par comptage au compteur à scintillation.

### b) Préparation des filtres

Après transformation des bactéries DH5α par un aliquot du mélange de ligation constituant la banque, 4000 clones Amp^{r} ont été obtenus sur boîtes LB + ampicilline, ce qui représente environ 3 fois la taille du génome.

Les colonies bactériennes sont transférées sur membrane de nylon (HYBOND N, AMERSHAM) et l'ADN dénaturé, selon la technique de GRUNSTEIN and HOGNESS [Proc. Natl. Acad. Sci. USA 72:3961-3965 (1975). Les membranes sont ensuite incubées 2 heures à 80°C.

### c) Hybridation

L'hybridation est réalisée à l'aide du kit "Rapid hybridization system multiprim" selon le protocole décrit par le fournisseur (AMERSHAM). Les membranes sont préhybridées dans le tampon d'hybridation pendant 15 mn à 65°C. La sonde dénaturée est rajoutée dans le tampon à raison de 10⁶ cpm/ml.

L'hybridation est réalisée à 65°C pendant la nuit.

Après hybridation, les membranes sont lavées :
- 2 fois 10 mn à 65°C avec 2 x SSPE, 0,1% SDS
- 1 fois 15 mn à 65°C avec 1 x SSPE, 0,1% SDS
- 2 fois 15 mn à 65°C avec 0,7 x SSPE, 0,1% SDS
(20 x SSPE : NaCl 3,6 M ; NaH₂ PO₄ 0,2 M ; EDTA 0,02 M)

Les membranes sont soumises à une autoradiographie de 12 heures à -80°C avec un film XRAY, (FUJI).

A l'issue de l'hybridation un clone positif appelé pG4 a été obtenu. Le plasmide a été extrait de ce clone par miniprep (MANIATIS, 1989, référence précitée ) et analysé par digestion par des enzymes de restriction. Le plasmide pG4 contient un insert d'ADN de *L. casei* de 3,5 kb.

La production de lactate dans *E. coli* a été déterminée pour le clone pG4. Après croissance de pG4 dans 10 ml de LB contenant 1% de glucose et de l'ampicilline (50 mg/ml), à 37°C, une production de lactate a été mise en évidence par dosage enzymatique à l'aide du kit L.lactate (BOEHRINGER).

Ceci confirme que le gène de L.LDH de *L. casei* est présent en entier sur le fragment de 3.5 kb isolé.

### EXEMPLE 2. - MODIFICATION DU GENE PAR MOTAGENESE

La séquence du gène LDH de *L. casei* a été récemment publiée [KIM et al., Appl. Environ. Microbiol. 57:2413-2417, (1991).

Il a été vérifié que la carte de restriction déduite de la séquence publiée était identique à celle d'un fragment central de l'insert de 3.5 kb du plasmide pG4.

Le codon d'initiation de la traduction du gène LDH de *L. casei* étant GTG, codon non utilisé par *Saccharomyces cerevisiae* comme codon d'initiation, GTG a été remplacé en ATG par mutagénèse.

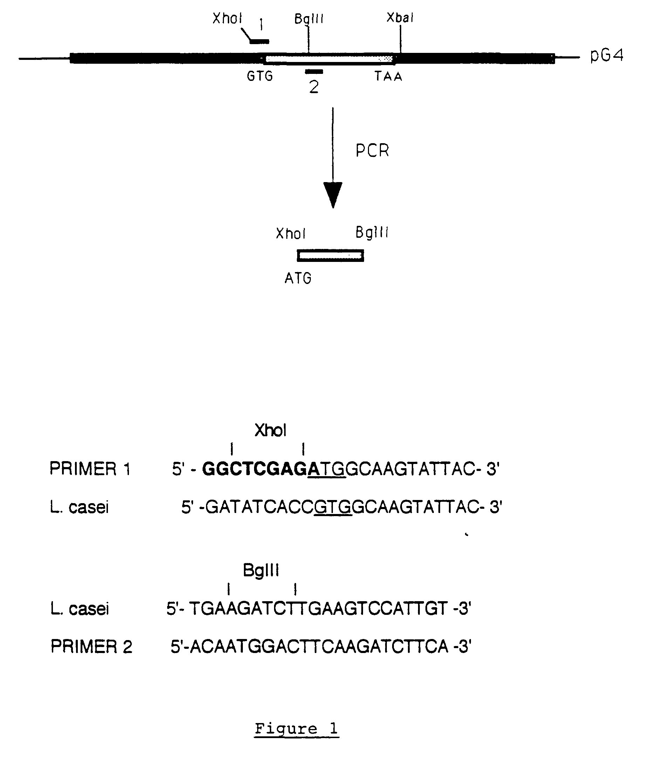

La stratégie de mutagénèse détaillée est résumée sur la figure 1.

### 1. Obtention du fragment mutagénéisé

L'insert de 3.5 kb contenu dans le plasmide pG4 est indiqué sur la figure 1, ainsi que la région codante du gène LDH (codon start GTG, codon stop TAA).

Le remplacement du GTG en ATG a été effectué par amplification PCR d'un fragment 5' du gène à partir du plasmide pG4 et à l'aide de deux amorces, dont la position et la séquence sont indiquées sur la figure 1 :
- L'amorce 1 est constituée de 13 bases complémentaires de la région codante en 5' et de 9 bases différentes : l'une étant un A de manière à remplacer le GTG en ATG, les 8 autres permettant la création d'un site XhoI en 5' du codon d'initiation.
- L'amorce 2 est constituée de 22 bases complémentaires d'une région interne à la région codante, comprenant le site BglII présent dans le gène.

Ces 2 amorces permettent l'amplification d'un fragment de 335 bases.

L'amplification a été réalisée de la manière suivante :

| | |
|---|---|
| Amorce 1 | 4 µl (20 pmoles) |
| Amorce 2 | 4 µl (20 pmoles) |
| Taq Buffer 10x | 10 µl |
| Taq 5u/µl | 0,5 µl |
| dNTP (2 mM) | 10 µl |
| pG4a | 10 µl 100 ng |
| Eau | 70,5 µl |

Conditions d'amplification : 30 secondes à 94°C, 30 secondes à 55°C, 1 mn à 72°C pendant 30 cycles.

La taille du fragment d'amplification a été vérifiée (335 bases) par analyse d'un aliquot sur gel d'agarose 1,5%.

1,5 µl du produit d'amplification ont été digérés par XhoI-BglII et le fragment digéré a été sous-cloné dans le plasmide pBSLDH₃ comme décrit ci-dessous (2.b).

### 2. Reconstruction du gène

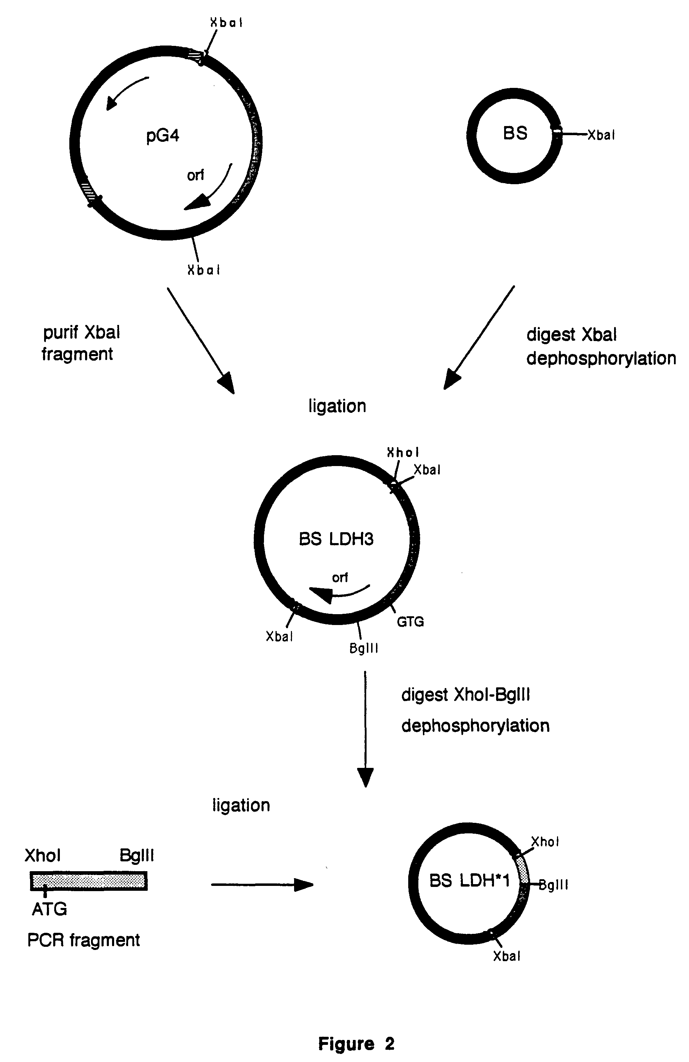

Les différentes étapes de cette construction sont schématisées sur la figure 2.

### a) Construction du plasmide pBSLDH3

Le fragment XbaI (2,2 kb) limité par un site en aval du codon stop TAA et un site dans le polylinker du plasmide pG4 a été isolé, par digestion XbaI de pG4, séparation des fragments sur gel d'agarose à bas point de fusion NIUSIEVE (FMC), et découpe de la bande de 2,2 kb.

Ce fragment a été sous cloné dans le plasmide pBS (pBluescript II SK+, STRATAGENE) par ligation de 200 ng de fragment XbaI (dans NIUSIEVE chauffé à 65°C) à 50 ng de plasmide pBS digéré par XbaI et déphosphorylé. Le plasmide recombinant obtenu est dénommé pBSLDH₃.

### b) Introduction du fragment modifié

Le plasmide pBSLDH₃ a été digéré par BglII (dans la région codante) et XhoI (site du polylinker), puis déphosphorylé.

100 ng de fragment amplifié par PCR et digéré par BglII- XhoI (comme indiqué en 1 ci-dessus), ont été ligués à 50 ng de pBSLDH₃ ainsi traité.

Le plasmide pBSLDH*₁ obtenu possède la région codante du gène LDH reconstituée, avec un codon ATG comme codon d'initiation, limitée par un site XhoI immédiatement en amont de l'ATG et par un site XbaI immédiatement en aval du codon stop TAA.

Le fragment XhoI-BglII de ce plasmide a été séquencé afin de vérifier d'une part le remplacement du GTG en ATG et d'autre part qu'aucune erreur n'a été commise par la Taq polymérase lors de l'amplification.

### EXEMPLE 3. - INTRODUCTION DU GENE MODIFIE DANS DES VECTEURS D'EXPRESSION

Afin d'obtenir une expression du gène LDH de *L. casei* mutagénéisé dans la levure, la région codante comprenant le codon ATG créé a été placée sous contrôle d'éléments régulateurs de levure, dans un vecteur navette levure/*E. coli.*

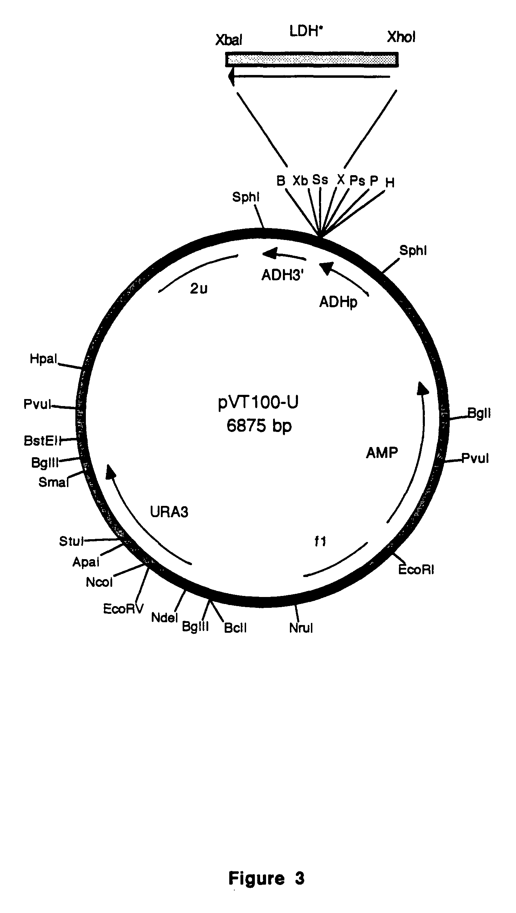

### 1) Introduction du gène LDH sur le plasmide multicopie pVT100-U (Figure 3)

Le plasmide d'expression qui a été utilisé est le plasmide pVT100-U contenant l'origine 2 µ, le marqueur URA3 et les éléments régulateurs forts ADH (promoteur et terminateur) ainsi que les éléments bactériens (origine et gène de résistance à l'ampicilline).

Ce plasmide a été décrit par VERNET et al. [Gene 52:225-233, (1987)].

Le fragment XhoI-XbaI du plasmide PBSLDH*₁ a été isolé par digestion XhoI-XbaI et séparation des fragments sur gel d'agarose à bas point de fusion NIUSIEVE. Le fragment XhoI-XbaI (1 kb) correspondant au gène a été découpé.

100 ng de ce fragment ont été ligués à 50 ng de vecteur pVT-100-U digéré par XbaI et XhoI (sites présents dans le polylinker), et déphosphorylé.

Le vecteur recombinant pVT-100-ULDH* a été obtenu.

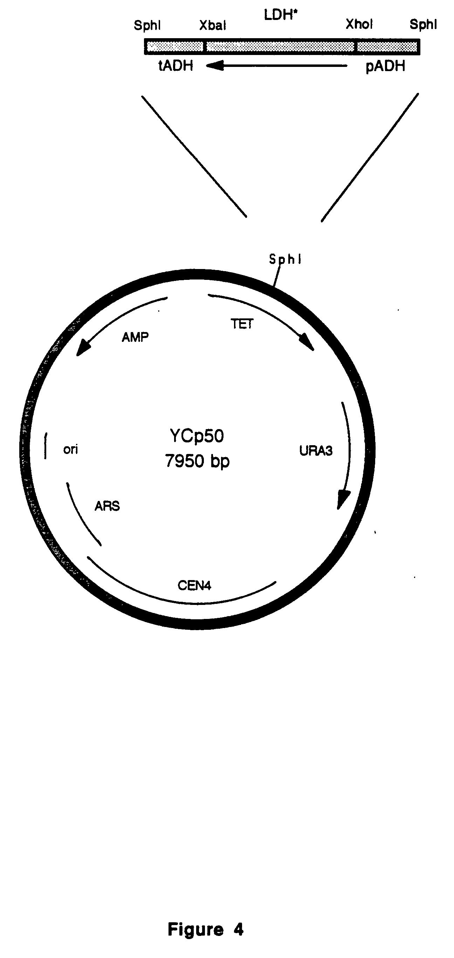

### 2) Introduction du gène LDH dans le plasmide monocopie YCp50 (Figure 4)

Le plasmide monocopie centromérique YCp50 a été décrit par ROSE [ S.L. BERGER and A.R. KIMMEL (Ed), Academic Press, 481-504 (1987)].

Ce vecteur porte une séquence ARS et une séquence centromérique et le marqueur URA3, ainsi que les éléments bactériens (origine de réplication et résistance à l'ampicilline).

Le fragment SphI de pVT 100-U-LDH* contenant la cassette d'expression pADH-LDH*-tADH a été isolé par digestion SphI et séparation des fragments sur gel d'agarose à bas point de fusion NIUSIEVE. Le fragment SphI contenant la cassette d'expression (1,7 kb) a été découpé. 100 ng de ce fragment ont été ligués à 50 ng de vecteur YCp50 digéré par SphI et déphosphorylé. Le vecteur recombinant YCp50-LDH* a été obtenu.

### EXEMPLE 4 - TRANSFORMATION DE LA LEVURE

La souche de levure *Saccharomyces cerevisiae* SCV5M a été tranformée par le plasmide pVT100-U-LDH* d'une part, et le plasmide YCp50-LDH* d'autre part .

La souche SCV5M a été déposée le 18 Juin 1992 auprès de la Collection Nationale de Cultures de Microorganismes, tenue par l'Institut Pasteur, sous le numéro I-1222. Il s'agit d'une souche de laboratoire haploïde, auxotrophe pour l'uracile (ura3), MAT a, dérivée d'une souche oenologique. Cette souche est capable de développer une fermentation en conditions oenologiques comparable à celle des souches industrielles.

La méthode de transformation utilisée est celle de l'acétate de lithium décrite par GIETZ et SCHIESTL [ Yeast, 7:253-263, (1991)].

Le milieu sélectif utilisé est du YNB (Yeast nitrogen base 7 g/l DIFCO, glucose 20 g/l). L'absence d'uracile permet de conserver une pression de sélection pour le plasmide.

### EXEMPLE 5 - ESSAIS DE FERMENTATION

### 1) Plasmide multicopie pVT100-U-LDH*

Les essais de fermentation ont été réalisés avec les souches suivantes :
- V5/pVT100-U : souche SCV5M transformée par le plasmide pVT100-U sans insert, à titre de témoin.
- V5/pVT100-U-LDH* : souche SCV5M transformée par le plasmide multicopie pVT100-U contenant le gène LDH modifié. Plusieurs transformants ont été testés séparément.

Les fermentations ont été réalisées sur milieu synthétique minimum YNB (yeast nitrogen base 7 g/l DIFCO) contenant 50 g/l de glucose, et tamponné à pH 5.1 par 6.3 g/l d'acide citrique et NaOH.

Des précultures des souches V5/pVT100-U et V5/pVT100-U-LDH* ont été réalisées pendant 20 heures dans 10 ml de milieu à 28°C.

Les cultures ont été réalisées dans 50 ml, par ensemencement de 7 x 10⁵ cellules/ml à partir des précultures. Le nombre de cellules a été déterminé sur un appareil de type COULTER COUNTER modèle ZBI.

Les cultures ont été incubées à 28°C, avec une agitation intermittente par barreau aimanté.

Le pH initial a été mesuré : 5 pour V5/pVT100-U et 4.9 pour V5/pVT100-U-LDH*.

Des prélèvements de 1 ml ont été réalisés à intervalles réguliers pour déterminer :
- le nombre de cellules, par comptage cellulaire (COULTER COUNTER) ;
- le pH du milieu de culture ;
- la concentration du milieu de culture en glucose, en éthanol et en lactate, par dosage enzymatique (kits de dosage BOHRINGHER ;
- l'activité spécifique lactate-déshydrogénase ; cette activité a été déterminée sur des extraits bruts de cellules obtenus comme suit : 10⁸ cellules sont prélevées, centrifugées 5 minutes à 6000 rpm, et lavées dans 5 ml de tampon acétate 80 mM pH 5.5 (Tampon acétate O.2M:Na acétate 2,73 g, dans 100 ml d'eau ; pH 5.5 avec de l'acide acétique). Le culot cellulaire est repris dans 0.5 ml du même tampon. Les cellules sont broyées sur vortex à l'aide de billes de verre, 4 fois 1 minute, au froid. Le broyat est centrifugé 5 minutes à 15000 rpm, et le surnageant récupéré est utilisé comme extrait brut. Le dosage de la LDH est réalisé comme décrit par HENSEL et al., [Arch. Microbiol. 112, 81-93 (1977)]. L'activité LDH est exprimée en U/mg de protéines de l'extrait

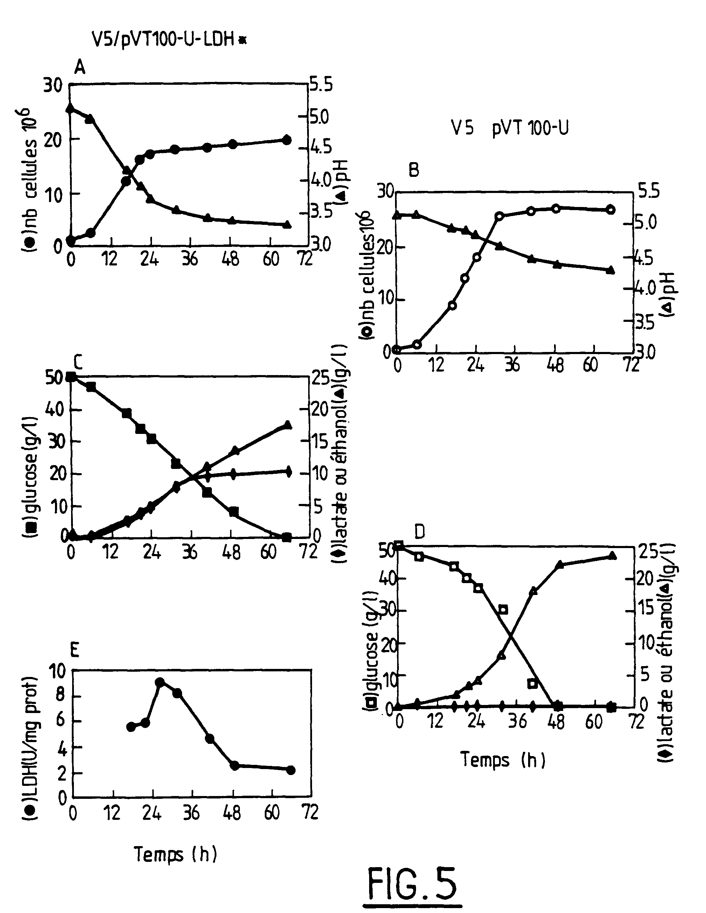

Les résultats des dosages effectués sont les suivants :
- Production d'éthanol et de lactate :
   La production de lactate de V5/pVT100-U-LDH* varie, selon les transformants testés, entre 6 et 25 g/l de lactate, avec une quantité produite de l'ordre de 10 g/l pour la majeure partie d'entre eux ; les résultats expérimentaux détaillés ci-après et illustrés par la figure 5 ont été obtenus réalisées avec un transformant V5/pVT100-U-LDH* produisant environ 10 g/l de lactate. Alors que la souche témoin produit de l'éthanol mais ne montre pas de production détactable de lactate (fig. 5D), le transformant produit simultanément du lactate et de l'éthanol (fig. 5C), ce qui correspond approximativement à la dégradation de 25 à 30% du glucose présent dans le milieu de culture en lactate.
   La production simultanée de lactate et d'éthanol est, de plus, mise en évidence pendant toute la phase exponentielle de croissance et au début de la phase stationnaire. Au cours de la phase stationnaire, la production de lactate s'arrête.
- Croissance cellulaire :
   La croissance du transformant V5/pVT100-U-LDH* (fig. 5 A) est comparable à celle de la souche témoin (fig. 5B). L'arrêt de la croissance (apparition de la phase stationnaire) se produit toutefois plus tôt pour la souche transformée que pour le témoin, et le nombre final de cellules est inférieur d'environ 20% à celui observé pour la souche témoin.
- Mesure du pH du milieu de culture
   L'arrêt de la croissance peut être expliqué par l'acidification importante du milieu de culture. On observe en effet une baisse de pH beaucoup plus forte dans le cas de la souche transformée (fig. 5A) que dans le cas de la souche témoin (fig 5B). Cette acidification importante est parfaitement corrélée à la production de lactate observée.
- Dosage de l'activité LDH :
   Les résultats obtenus sur l'extrait brut de la souche transformée (fig. 5E) montrent que l'activité spécifique de la LDH est maximale quand les cellules entrent en phase stationnaire, puis diminue au cours de cette même phase.
- Autres milieux de culture :
   Les résultats obtenus sur milieu YNB ont en outre été confirmés sur jus de raisin (185 g/l glucose) et jus de pomme (93 g/l glucose).
   L'obtention d'une production mixte lactate-éthanol peut donc être réalisée sur différents milieux (synthétiques, minimum ou complets, naturels) contenant des concentrations, en glucose variable, et quel que soit le pH de départ. La gamme de température utilisable est celle permettant la croissance des levures (approximativement 14-35°C).

### 2) Plasmide monocopie YCp5O-LDH*

Des essais de fermentation ont été réalisés avec la souche V5/YCp5O-LDH* sur milieu YNB contenant 50 g/l de glucose tamponné à pH 5.1. Une production de lactate de l'ordre de 1 g/l a été obtenue pour cette souche.

L'activité spécifique de la LDH, déterminée comme précédemment décrit, présente des variations similaires à celles observées avec les transformants multicopie. Par contre, l'activité maximale, observée en fin de phase exponentielle-début de phase stationnaire, est de l'ordre de 1.5 U/mg protéines, soit 7 fois moins que l'activité maximale obtenue avec le transformant multicopie (10 U/mg prot).

La production de lactate par le transformant monocopie est donc en corrélation avec le niveau d'activité spécifique de la LDH.

Ceci montre que la production de lactate peut être modulée notamment en fonction du nombre de gènes LDH introduits dans la levure.

Ainsi que cela ressort de ce qui précède, l'Invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée de la présente Invention.

## Revendications

1. Souches de levure **caractérisées en ce qu'**elles contiennent au moins une copie d'un gène codant pour une LDH de bactérie lactique, sous contrôle de séquences régulant l'expression dudit gène dans la levure.

2. Souches selon la Revendication 1, **caractérisées en ce que** lesdites levures appartiennent au genre *Saccharomyces.*

3. Souches selon l'une quelconque des Revendications 1 ou 2, **caractérisées en ce que** le gène exprimé est celui de la LDH de *Lactobacillus casei.*

4. Cassette d'expression **caractérisée en ce qu'**elle comprend le gène d'une LDH de bactérie lactique associé à des séquences de régulation actives dans la levure.

5. Vecteurs d'expression, **caractérisés en ce qu'**ils comprenent une cassette d'expression selon la Revendication 4, et utilisables pour l'obtention des souches de levure transformées selon la Revendication 1.

6. Vecteurs selon la Revendication 5, **caractérisés en ce qu'**il s'agit de vecteurs navette, possédant également une origine de réplication bactérienne et un marqueur de sélection dans une bactérie.

## Patentansprüche

1. Hefestämme, **dadurch gekennzeichnet, daß** sie wenigstens eine Kopie eines für eine LDH von Milchsäurebakterien codierenden Gens unter Kontrolle von Sequenzen enthalten, welche die Expression dieses Gens in der Hefe regulieren.

2. Stämme nach Anspruch 1, **dadurch gekennzeichnet, daß** die Hefen zur Gattung *Saccharomyces* gehören.

3. Stämme nach irgendeinem der Ansprüche 1 oder 2, **dadurch gekennzeichnet, daß** das exprimierte Gen dasjenige für die LDH von *Lactobacillus casei* ist.

4. Expressionskassette, **dadurch gekennzeichnet, daß** sie das Gen für eine LDH von Milchsäurebakterien in Verbindung mit in der Hefe aktiven Regulationssequenzen umfaßt.

5. Expressionsvektoren, **dadurch gekennzeichnet, daß** sie eine Expressionskassette nach Anspruch 4 umfassen und zum Erhalt der transformierten Hefestämme nach Anspruch 1 verwendbar sind.

6. Vektoren nach Anspruch 5, **dadurch gekennzeichnet, daß** es sich um Shuttle-Vektoren handelt, die sowohl einen bakteriellen Replikationsursprung als auch einen Selektionsmarker in einem Bakterium besitzen.

## Claims

1. Yeast strains **characterized in that** they comprise at least one copy of a gene coding for a lactic acid bacterium lactic dehydrogenase (LDH), under the control of sequences regulating the expression of said gene in yeast.

2. Strains according to claim 1, **characterized in that** said yeast is of the genus *Saccharomyces.*

3. Strains according to any one of claims I or 2, **characterized in that** the expressed gene is the gene of *Lactobacillus casei* LDH.

4. Expression cassette **characterized in that** it comprises the gene of a lactic acid bacterium LDH associated with regulatory sequences which are active in yeast.

5. Expression vectors, **characterized in that** they comprise an expression cassette according to claim 4, for use in producing the transformed yeast strains according to claim 1.

6. Vectors according to claim 5, **characterized in that** they are shuttle vectors, further comprising a bacterial origin of replication and a marker which is selectable in a bacterium.
